(19) Europäisches Patentamt
European Patent Office
Office européen des brevets

(11) Veröffentlichungsnummer: **0 305 805**
**A2**

(12) **EUROPÄISCHE PATENTANMELDUNG**

(21) Anmeldenummer: **88113327.6**

(22) Anmeldetag: **17.08.88**

(51) Int. Cl.$^4$: **C08L 101/00 , C08L 75/04 , C08G 18/61**

(30) Priorität: **26.08.87 DE 3728395**
**15.04.88 DE 3812482**

(43) Veröffentlichungstag der Anmeldung:
**08.03.89 Patentblatt 89/10**

(84) Benannte Vertragsstaaten:
**AT BE CH DE ES FR GB GR IT LI LU NL SE**

(71) Anmelder: **BAYER AG**
**Konzernverwaltung RP Patentabteilung**
**D-5090 Leverkusen 1 Bayerwerk(DE)**

(72) Erfinder: **Jansen, Bernhard, Dr.**
**Roggendorfstrasse 65**
**D-5000 Köln 80(DE)**
Erfinder: **Müller, Hanns Peter, Dr.**
**Weizenfeld 36**
**D-5060 Bergisch Gladbach 2(DE)**
Erfinder: **Richter, Roland, Dr.**
**Roggendorfstrasse 53**
**D-5000 Köln 80(DE)**
Erfinder: **Mayer, Wolfram, Dr.**
**Auf dem Heidchen 17**
**D-5068 Odenthal-Glöbusch(DE)**
Erfinder: **Schlak, Ottfried**
**Carl-Duisberg-Strasse 331**
**D-5090 Leverkusen(DE)**

(54) **Wasserhärtende Polymerzubereitung.**

(57) Wasserhärtende Polymerzubereitungen für Konstruktionsmaterialien enthalten als Additive Polysiloxane mit Polyethergruppen.

EP 0 305 805 A2

## Wasserhärtende Polymerzubereitung

Die Erfindung betrifft wasserhärtende Polymerzubereitungen für Konstruktionsmaterialien, insbesondere für medizinische Stützverbände oder technische Vorrichtungen, die als Additive Polysiloxane mit Polyethergruppen enthalten, ein Verfahren zu ihrer Herstellung und ihre Verwendung.

Die erfindungsgemäßen Konstruktionsmaterialien bestehen im allgemeinen aus einer Trägerschicht, die mit einer wasserhärtenden Polymerzubereitung beschichtet und/oder imprägniert ist.

Im allgemeinen können die erfindungsgemäßen Konstruktionsmaterialien zur Versteifung, Formgebung und Abdichtung im medizinischen oder technischen Bereich verwendet werden. Im medizinischen Bereich werden sie bevorzugt als Gipsersatz eingesetzt.

Die erfindungsgemäßen Konstruktionsmaterialien können aber auch zur Herstellung von Behältern, Filtern, von Rohren, zum Verbinden von Konstruktionselementen, zur Fabrikation von dekorativen oder künstlerischen Artikeln, zu Versteifungszwecken oder als Füll- bzw. Dichtungsmaterial für Fugen und Hohlräume eingesetzt werden.

Konstruktionsmaterialien, die aus einem flexiblen Träger bestehen, der mit einem wasserhärtenden Reaktivharz beschichtet und/oder getränkt ist, sind bereits bekannt. Beispielsweise sei die DE-A-23 57 931 genannt, in der Konstruktionsmaterialien aus flexiblen Trägern, wie Gewirken, Geweben oder Vliesen, beschrieben werden, die mit wasserhärtenden Reaktivharzen, wie Isocyanaten oder durch Isocyanatgruppen modifizierte Prepolymere, beschichtet oder getränkt sind.

Die aus der DE-A-23 57 931 bekannten wasserhärtenden Polymerzubereitungen und die späteren Nachfolgeentwicklungen haben den Nachteil, daß sie sich nur schlecht modellieren lassen, insbesondere während der Aushärtungsphase, da sie dann eine starke Klebrigkeit entwickeln.

Dieses Problem versucht man in der EP-A-02 21 669 durch Zusatz von Schmiermitteln zu lösen. Die in der EP-A-02 21 660 beschriebenen Flächengebilde, z.B. orthopädische Binden, bestehen aus einem mit einer Polymerzubereitung beschichteten Träger, der oberflächlich zum größten Teil mit einem Schmiermittel beschichtet ist. Bei den Schmiermitteln handelt es sich um Verbindungen mit hydrophilen Gruppen, die kovalent an das Polymer gebunden sind, oder um Additive, die inkompatibel mit dem Polymer sind. Die Menge an Schmiermittel wird so hoch gewählt, daß sie einen kinetischen Reibungskoeffizienten der beschichteten Flächengebilde von weniger als 1,2 bewirken. Die so behandelten Flächengebilde weisen eine geringe Klebrigkeit auf. Als unverträgliche Additive werden unter anderem auch Polysiloxane eingesetzt (Seite 8, Zeile 28 bis Seite 10, Zeile 8). Diese bekannten hydrophoben Polysiloxane sind mit dem Polymerharz nicht mischbar, wodurch ein umständliches separates Auftragen auf den bereits beschichteten Träger erforderlich ist.

Es wurden wasserhärtende Polymerzubereitungen für Konstruktionsmaterialien gefunden, die als Additive Polysiloxane der Formel

$$R^1{}_3Si\text{-}O\text{-}[A]_m\text{-}SiR^1{}_3 \quad (I) \qquad \text{und/oder} \qquad R^2\text{-}Y\text{-}X\text{-}\left[\;A\;\right]_m\text{—}\underset{\underset{\displaystyle R^1}{|}}{\overset{\overset{\displaystyle R^1}{|}}{Si}}\text{-}X\text{-}Y\text{-}R^2 \quad (II)$$

enthalten, in der

$R^1$ für Niederalkyl steht,

m für eine mittlere Zahl von 1 bis 20 steht und

Y für einen Polyetherrest mit 1 bis 15 Ethylenoxid-Gruppen steht,

A für eine Polysiloxankette mit Strukturelementen der Formeln

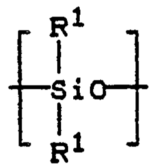 und/oder 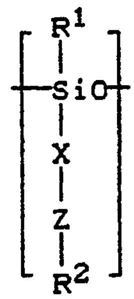 steht,

worin
$R^1$ die obengenannte Bedeutung hat,
X für einen Niederalkylenrest steht,
Z für einen Polyetherrest mit Ethylenoxid- und/oder Propylenoxid-Gruppen steht, wobei die mittlere Zahl der Ethylenoxid- und/oder Propylenoxid-Gruppen im Bereich von 10 bis 100 liegt, und
$R^2$ Wasserstoff und/oder Niederalkyl bedeuten.

Die erfindungsgemäßen Additive sind kompatibel mit den Polymerzubereitungen und vermischen sich völlig mit diesen. Sie zeigen eine ausgezeichnete Lagerstabilität und entmischen sich auch nach mehr als 12 Monaten Lagerung nicht. Dadurch ist es möglich, die erfindungsgemäßen Additive bereits bei der Herstellung der wasserhärtenden Polymerzubereitung beizumischen, was gegenüber den bekannten Materialien einen Verarbeitungsschritt einspart. Überraschenderweise weisen auch die mischbaren erfindungsgemäßen Additive bei der Verarbeitung eine geringere Klebrigkeit auf. Die Zubereitungen lassen sich während der Aushärtzeit leicht modellieren, jedoch sind sie nicht ganz so schlüpfrig wie die Produkte der EP 221 669, so daß insbesondere während der Verarbeitungsphase das Produkt besser gehandhabt werden kann.

Im Rahmen der vorliegenden Erfindung können im allgemeinen die Substituenten der Polysiloxane die folgende Bedeutung haben:
Niederalkyl kann im allgemeinen einen geradkettigen oder verzweigten Kohlenwasserstoffrest mit 1 bis etwa 6 Kohlenstoffatomen bedeuten. Beispielsweise seien Methyl, Ethyl, Propyl, Isopropyl, Butyl, Isobutyl, Pentyl, Isopentyl, Hexyl und Isohexyl genannt. Bevorzugt werden Methyl und Ethyl. Insbesondere bevorzugt wird Methyl.

Niederalkylen kann im allgemeinen einen zweibindigen, geradkettigen oder verzweigten Kohlenwasserstoffrest mit 1 bis etwa 6 Kohlenstoffatomen bedeuten. Beispielsweise sei Methylen, Ethylen, Propylen, Isopropylen, Butylen, Isobutylen, Pentylen, Isopentylen, Hexylen und Isohexylen genannt. Bevorzugt wird Methylen, Ethylen und Propylen.

Der Polyetherrest Y besteht im allgemeinen aus 1 bis 15 Ethylenoxid-, bevorzugt 2 bis 10, Ethylenoxid-Gruppen.

Der Polyetherrest (Z) besteht im allgemeinen aus Ethylenoxid- und/oder Propylenoxid-Gruppen. Bevorzugt besteht er aus Ethylenoxid- und Propylenoxid-Gruppen. Die mittlere Gesamtzahl der Ethylenoxid- und Propylen-Gruppen liegt im allgemeinen im Bereich von etwa 10 bis etwa 100, bevorzugt von 15 bis 80. Für den bevorzugten Fall, daß der Polyetherrest aus Ethylenoxid- und Propylenoxid-Gruppen besteht, beträgt im allgemeinen das Gewichts-Verhältnis Ethylenoxid zu Propylenoxid 20:80 % bis 80:20 %, bevorzugt 30:70 % bis 70:30 %.

Die Ethylenoxid- und Propylenoxid-Gruppen können statistisch, alternierend oder in Blöcken geordnet sein. Bevorzugt ist eine statistische Verteilung.

Die Zahl m gibt die mittlere Zahl der Siloxangruppen (A) an. Sie liegt im allgemeinen im Bereich von 1 bis 20, bevorzugt von 2 bis 16.

Die Siloxangruppen sind bevorzugt durch das Strukturelement

$-X-Z-R^2$

in dem
X, Z und $R^2$ die obengenannte Bedeutung haben,
substituiert.

Der Siloxananteil beträgt bei Formel (I) im allgemeinen 5 bis 30 Gew.-%, bevorzugt 7 bis 25 Gew.-%, und der Anteil der Polyether ($-X-Z-R^2$) beträgt 70 bis 95 Gew.-%, bevorzugt 75 bis 93 Gew.-%, jeweils bezogen auf das Polysiloxan. Bei Formel (II) liegt der Siloxananteil über 50 %.

Das Molekulargewicht liegt bevorzugt im Bereich von 1.200 bis 3000 [g/Mol].

Die erfindungsgemäßen wasserhärtenden Polymerzubereitungen für Konstruktionsmaterialien weisen einen kinetischen Reibungskoeffizienten auf, der nur 0,1 bis 0,3 geringer ist als ohne die entsprechenden Additive.

Es ist möglich, daß die erfindungsgemäßen Additive noch teilweise Hydroxylgruppen aufweisen ($R^2$ = Wasserstoff). Der Anteil an Hydroxylgruppen wird durch die Hydroxyl-Zahl charakterisiert. Sie liegt im allgemeinen im Bereich von 0 bis 80, bevorzugt von 20 bis 70, [mgKOH/g]. In diesem Fall handelt es sich um Additive mit hydrophobem Charakter, die zum Teil kovalent an das Polymer gebunden sind, Auch diese besitzen entgegen dem Vorurteil der EP 221 669 noch einen positiven Effekt bezüglich Reduktion der Klebrigkeit.

Bevorzugt können die erfindungsgemäßen wasserhärtenden Polymerzubereitungen als Additive Polysiloxane der Formel (I) und/oder der Formel (II) enthalten

in der

$R^1$ für Methyl oder Ethyl steht,

m für die mittlere Zahl an Siloxangruppen im Bereich von 2 bis 16 steht,

Y für einen Polyetherrest mit 1 bis 15 Ethylenoxid-Gruppen steht,

A für eine Polysiloxankette mit Strukturelementen der Formeln

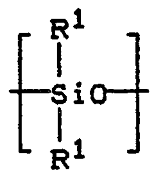 und/oder 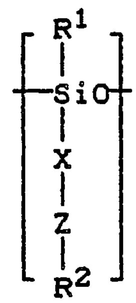 steht,

worin

$R^1$ die obengenannte Bedeutung hat,

X für Alkylen ($C_1$ bis $C_4$) steht,

Z für einen Polyetherrest mit Ethylenoxid- und Propylenoxid-Gruppen steht, wobei die mittlere Zahl der Ethylenoxid- und Propylenoxid-Gruppen im Bereich von 10 bis 100 liegt, und

$R^2$ Wasserstoff und/oder Alkyl ($C_1$ bis $C_4$) bedeutet,

wobei das Verhältnis der Ethylenoxid- zu Propylenoxid-Gruppen 30:70 % bis 70:30 % beträgt,

wobei die Hydroxy-Zahl im Bereich von 0 bis 80 [mgKOH/g] liegt.

Insbesonders bevorzugt können die erfindungsgemäßen wasserhärtenden Polymerzubereitungen als Additive Polysiloxane der Formel (I) und/oder Formel (II) enthalten

in der

$R^1$ für Methyl oder Ethyl steht,

m für die mittlere Zahl an Siloxangruppen im Bereich von 2 bis 16 steht und

Y für einen Polyetherrest von 2 bis 10 Ethylenoxid-Gruppen,

A für eine Polysiloxankette mit Strukturelementen der Formeln

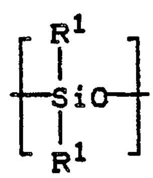 und/oder 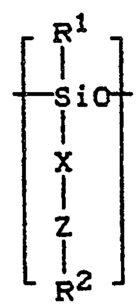 steht,

worin

$R^1$ die obengenannte Bedeutung hat,
X für Alkylen ($C_1$ bis $C_4$) steht,
Z für einen Polyetherrest mit Ethylenoxid- und Propylenoxid-Gruppen steht, wobei die mittlere Zahl der Ethylenoxid- und Propylenoxid-Gruppen im Bereich von 15 bis 80 liegt, und
$R^2$ Wasserstoff und/oder Alkyl ($C_1$ bis $C_4$) bedeutet,
wobei das Verhältnis der Ethylenoxid- zu Propylenoxid-Gruppen 30:70 % bis 70:30 % beträgt,
wobei die Hydroxy-Zahl im Bereich von 20 bis 70 [mgKOH/g] liegt.

Verbindungen der Formel (II) werden besonders bevorzugt.

Die Gruppe der als Additive verwendeten Polysiloxane ist an sich bekannt (Herstellung gemäß DE-A 1 595 730 und DE-A 3 133 869); sie werden als Stabilisatoren für Polyurethanschäume eingesetzt (DE-A 2 533 074).

Es ist als ausgesprochen überraschend anzusehen, daß derartige Verbindungen, die in der PUR-Technik als Schaumstabilisatoren eingesetzt werden, beim erfindungsgemäßen Einsatz nicht in dieser Weise wirken, sondern sich wie Gleitmittel verhalten. Obwohl beim Abbinden der Polymethanprepolymere $CO_2$ abgespalten wird, härten die erfindungsgemäßen Zubereitungen ohne Schaumbildung aus. Dieser Effekt war nicht zu erwarten.

Die erfindungsgemäßen wasserhärtenden Polymerzubereitungen enthalten im allgemeinen 0,1 bis 10 Gew.-%, bevorzugt 0,3 bis 8 Gew.-%, Polysiloxane, bezogen auf das Polymerharz, wobei der kinetische Reibungskoeffizient nur 0,1 bis 0,3 kleiner ist als der ohne diese Menge der entsprechenden Additive.

Selbstverständlich ist es möglich, daß die erfindungsgemäßen wasserhärtenden Polymerzubereitungen mehrere der genannten erfindungsgemäßen Polysiloxane als Additive enthalten.

Wasserhärtende Polymerzubereitungen sind bevorzugt Harze auf Polyurethan- oder Polyvinylharz-Basis.

Besonders bevorzugt sind Polyisocyanate bzw. Prepolymere mit freien Isocyanat-Gruppen (Polyurethane).

Als wasserhärtende Polyisocyanate bzw. Polyurethane kommen erfindungsgemäß alle an sich bekannten organischen Polyisocyanate in Frage, d.h. beliebige Verbindungen bzw. Gemische von Verbindungen, die pro Molekül mindestens zwei organisch gebundene Iso cyanatgruppen ausweisen. Hierzu gehören sowohl niedermolekulare Polyisocyanate mit einem unter 400 liegendem Molekulargewicht als auch Modifizierungsprodukte derartiger niedermolekularer Polyisocyanate mit einem aus der Funktionalität und dem Gehalt an funktionellen Gruppen berechenbaren, z.B. 400 bis 10.000, vorzugsweise 600 bis 8.000, und insbesondere 800 bis 5.000, betragenden Molekulargewicht. Geeignete niedermolekulare Polyisocyanate sind beispielsweise solche der Formel

$$Q\ (NCO)_n,$$

in der
n = 2 bis 4, vorzugsweise 2 bis 3,
und
Q einen aliphatischen Kohlenwasserstoffrest mit 2 bis 18, vorzugsweise 6 bis 10 C-Atomen,
einen cycloaliphatischen Kohlenwasserstoffrest mit 4 bis 15, vorzugsweise 5 bis 10 C-Atomen,
einen aromatischen Kohlenwasserstoffrest mit 6 bis 15, vorzugsweise 6 bis 13 C-Atomen,
oder einen araliphatischen Kohlenwasserstoffrest mit 8 bis 15, vorzugsweise 8 bis 13 C-Atomen,
bedeuten.

Geeignete derartige niedermolekulare Polyisocyanate sind z.B. Hexamethylendiisocyanat, 1,12-Dodecandiisocyanat, Cyclobutan-1,3-diisocyanat, Cyclohexan-1,3- und -1,4- diisocyanat sowie beliebige Gemische dieser Isomeren, 1-Isocyanato-3,3,5-trimethyl-5-isocyanatomethylcyclohexan, 2,4- und 2,6-Hexahydrotoluylendiisocyanat sowie beliebige Gemische dieser Isomeren, Hexahydro-1,3-und/oder -1,4-phenylendiisocyanat, Perhydro-2,4′-und/oder -4,4′-diphenylmethan-diisocyanat, 1,3- und 1,4-Phenylendiisocyanat, 2,4- und 2,6-Toluylendiisocyanat sowie beliebige Gemische dieser Isomeren, Diphenylmethan-2,4′- und/oder -4,4′-diisocyanat, Naphthylen-1,5-diisocyanat, Triphenylmethan-4,4′, 4″-triisocyanat oder Polyphenyl-polymethylenpolyisocyanate, wie sie durch Anilin-Formaldehyd-Kondensation und anschließende Phosgenierung erhalten werden.

Geeignete höhermolekulare Polyisocyanate sind Modifizierungsprodukte derartiger einfacher Polyisocyanate, d.h. Polyisocyanate mit z.B. Isocyanurat-, Carbodiimid-, Allophanat-, Biuret- oder Uretdion-Struktureinheiten, wie sie nach an sich bekannten Verfahren des Standes der Technik aus den beispielhaft genannten einfachen Polyisocyanaten der oben genannten allgemeinen Formel hergestellt werden können. Unter den höhermolekularen, modifizierten Polyisocyanaten sind insbesondere die aus der Polyurethanchemie bekannten Prepolymeren mit endständigen Isocyanatgruppen des Molekulargewichtsbereichs 400 bis 10.000, vorzugsweise 600 bis 8.000 und insbesondere 800 bis 5.000, von Interesse. Diese Verbindungen werden in an sich bekannter Weise durch Umsetzung von überschüssigen Mengen an einfachen Polyisocyanaten der beispielhaft genannten Art mit organischen Verbindungen mit mindestens zwei gegenüber Isocyanatgruppen reaktionsfähigen Gruppen, insbesondere organischen Polyhydroxylverbindungen hergestellt. Geeignete derartige Polyhydroxylverbindungen sind sowohl einfache mehrwertige Alkohole wie z.B. Ethylenglykol, Trimethylolpropan, Propandiol-1,2 oder Butandiol-1,2, insbesondere jedoch höhermolekulare Polyetherpolyole und/oder Polyesterpolyole der aus der Polyurethanchemie an sich bekannten Art mit Molekulargewichten von 600 bis 8.000, vorzugsweise 800 bis 4.000, die mindestens zwei, in der Regel 2 bis 8, vorzugsweise aber 2 bis 4 primäre und/oder sekundäre Hydroxylgruppen aufweisen. Selbstverständlich können auch solche NCO-Prepolymere eingesetzt werden, die beispielsweise aus niedermolekularen Polyisocyanaten der beispielhaft genannten Art und weniger bevorzugten Verbindungen mit gegenüber Isocyanatgruppen reaktionsfähigen Gruppen wie z.B. Polythioetherpolyolen, Hydroxylgruppen aufweisenden Polyacetalen, Polyhydroxypolycarbonaten, Hydroxylgruppen aufweisenden Polyesteramiden oder Hydroxylgruppen aufweisenden Copolymerisaten olefinisch ungesättigter Verbindungen erhalten worden sind. Zur Herstellung der NCO-Prepolymeren geeignete Verbindungen mit gegenüber Isocyanatgruppen reaktionsfähigen Gruppen, insbesondere Hydroxylgruppen, sind beispielsweise die in US 4 218 543, Kolonne 7, Zeile 29 bis Kolonne 9, Zeile 25 beispielhaft offenbarten Verbindungen. Bei der Herstellung der NCO-Prepolymeren werden diese Verbindungen mit gegenüber Isocyanatgruppen reaktionsfähigen Gruppen mit einfachen Polyisocyanaten der oben beispielhaft genannten Art unter Einhaltung eines NCO/OH-Äquivalentverhältnisses von >1 zur Umsetzung gebracht. Die NCO-Prepolymeren weisen im allgemeinen einen NCO-Gehalt von 2,5 bis 30, vorzugsweise 6 bis 25 Gew.-% auf. Hieraus geht bereits hervor, daß im Rahmen der vorliegenden Erfindung unter "NCO-Prepolymeren" bzw. unter "Prepolymeren mit endständigen Isocyanatgruppen" sowohl die Umsetzungsprodukte als solche als auch ihre Gemische mit überschüssigen Mengen an nicht umgesetzten Ausgangspolyisocyanaten, die oft auch als "Semiprepolymer" bezeichnet werden, zu verstehen sind.

Erfindungsgemäß besonders bevorzugte Polyisocyanatkomponenten sind die in der Polyurethanchemie üblichen technischen Polyisocyanate, d.h. Hexamethylendiisocyanat, 1-Isocyanato-3,3,5-trimethyl-5-isocyanatomethyl-cyclohexan (Isophorondiisocyanat, abgekürzt: IPDI), 4,4′-Diisocyanato-dicyclohexylmethan, 4,4′-Diisocyanatodiphenylmethan, dessen Gemische mit den entsprechenden 2,4′- und 2,2′-Isomeren, Polyisocyanatgemische der Diphenylmethanreihe wie sie durch Phosgenierung von Anilin/Formaldehyd-Kondensaten in an sich bekannter Weise gewonnen werden können, die Biuret- oder Isocyanuratgruppen aufweisenden Modifizierungsprodukte dieser technischen Polyisocyanate und insbesondere NCO-Prepolymere der genannten Art auf Basis dieser technischen Polyisocyanate einerseits und den beispielhaft genannten einfachen Polyolen und/oder Polyetherpolyolen und/oder Polyesterpolyolen andererseits, sowie beliebige Gemische derartiger Polyisocyanate. Isocyanate mit aromatisch gebundenen NCO-Gruppen sind erfindungsgemäß bevorzugt. Eine erfindungsgemäß besonders bevorzugte Polyisocyanat-Komponente stellt teilweise carbodiimidisiertes Diisocyanatodiphenylmethan dar, welches infolge Anlagerung von monomerem Diisocyanat an die Carbodiimid-Struktur auch Uretonimingruppen aufweist.

Die wasserhärtenden Polyurethane können an sich bekannte Katalysatoren enthalten. Insbesondere können dies tert. Amine sein, die die Isocyanat/Wasser-Reaktion und nicht eine Selbstreaktion (Trimerisierung, Allophanatisierung) katalysieren (DE-A 23 57 931). Als Beispiele seien genannt tert. aminhaltige Polyether (DE-A 26 51 089), niedermolekulare tert. Amine, wie

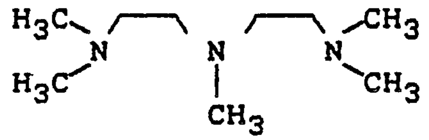

oder Dimorpholindiethylether oder Bis-(2,6-dimethylmorpholino)-diethylether (WO 86/01397). Der Gehalt an Katalysator bezogen auf den tert.-Stickstoff beträgt im allgemeinen 0,05 bis 0,5 Gew.-% bezogen auf das Polymerharz.

Wasserhärtende Polyvinylharze können beispielsweise Vinylverbindungen sein, die aus einem hydrophilen Prepolymer mit mehr als einer polymerisierbaren Vinylgruppe bestehen, in der ein fester, unlöslicher Vinyl-Redox Katalysator eingelagert ist, dessen einer Bestandteil von einer wasserlöslichen bzw. wasserdurchlässigen Hülle umkapselt ist. Ein solcher Redox Katalysator ist beispielsweise Natriumhydrogensulfit/Kupfer(II)sulfat, bei dem beispielsweise das Kupfersulfat mit Poly-2-hydroxyethylmethylacrylat verkapselt ist.

Polyvinylharze werden beispielsweise in der EP-A 01 36 021 beschrieben.

Die wasserhärtenden Polymerzubereitungen können an sich bekannte Zusatzmittel enthalten, wie z.B. Verlaufshilfsmittel, Thixotropiermittel, Entschäumer und andere bekannte Gleitmittel.

Weiterhin können die Kunststoffharze eingefärbt sein oder, falls erwünscht, UV-Stabilisatoren enthalten.

Als Zusatzmittel seien beispielsweise genannt: Polydimethylsiloxane, Calciumsilikate von Aerosil-Typ, Polywachse (Polyethylenglykole), UV-Stabilisatoren vom Ionol-Typ (DE-A 29 21 163), Farbpigmente, wie Ruß, Eisenoxide, Titandioxid oder Phthalocyanine.

Die insbesondere für Polyurethan-Prepolymere geeigneten Zusatzmittel sind im Kunststoff-Handbuch, Band 7, Polyurethane, Seiten 100 bis 109 (1983) beschrieben. Sie werden im allgemeinen in einer Menge von 0,5 bis 5 % (bezogen auf das Harz) zugesetzt.

Als Trägermaterialien kommen massive oder poröse Folien oder auch Schaumstoffe aus natürlichen oder synthetischen Materialien (z.B. Polyurethan), in erster Linie aber luftdurchlässige, flexible Flächengebilde auf textiler Basis in Betracht, vorzugsweise mit einem Flächengewicht von 20 bis 1.000 $g/m^2$, insbesondere von 30 bis 500 $g/m^2$. Als Flächengebilde seien beispielsweise genannt:

Trägermaterial

1. Textile Gewebe, Gewirke oder Gestricke eines Flächengewichts von 20 bis 400 $g/m^2$, vorzugsweise 40 bis 250 $g/m^2$, mit 25 bis 100 Maschenreihen pro 10 cm, vorzugsweise 30 bis 75 Maschenreihen pro 10 cm und 30 bis 90 Maschenstäbe pro 10 cm, vorzugsweise 40 bis 80 Maschenstäbe pro 10 cm. Das textile Gewebe, Gewirke oder Gestricke kann aus beliebigen natürlichen oder synthetischen Garnen hergestellt sein. Vorzugsweise werden Garne eingesetzt, die aus Baumwoll-, Polyester-, Polyacrylat-, Polyamid- oder Elasthan-Fasern bestehen bzw. aus Gemischen der vorgenannten. Besonders bevorzugt sind textile Träger aus den vorgenannten Garnen, die eine Dehnung in Längsrichtung von 10 bis 100 % und/oder in Querrichtung von 20 bis 300 % aufweisen.

2. Glasfasergewebe, -gewirke oder -gestricke eines Flächengewichts von 60 bis 500 $g/m^2$, vorzugsweise 100 bis 400 $g/m^2$, hergestellt aus Glasfasergarnen mit einem E-Modul von 7.000 bis 9.000 ($daN/mm^2$), und einer Fadenzahl von 3 bis 10, bevorzugt 5 bis 7 in Längsrichtung und mit einer Fadenzahl von 3 bis 10, bevorzugt 4 bis 6 in Querrichtung je Zentimeter Glasfasergewebe, die durch eine spezielle Art der Hitzebehandlung eine Längenelastizität von 10 bis 30 % besitzen, sind bevorzugt. Die Gewirke können sowohl geschlichtet als auch ungeschlichtet sein.

3. Nicht gebundene oder gebundene oder genadelte Vliese auf Basis von anorganischen und vorzugsweise organischen Fasern eines Flächengewichts von 30 bis 400 $g/m^2$, vorzugsweise 50 bis 200 $g/m^2$.

Für die Herstellung erfindungsgemäßer Konstruktionsmaterialien in Form von Schalen oder Schienen kommen auch Vliese mit Flächengewichten bis 1.000 $g/m^2$ in Betracht. Erfindungsgemäß geeignete Trägermaterialien werden beispielsweise auch in US 4 134 397, US 3 686 725, US 3 882 857, DE-A 3 211 634 und EP-A 61 642 beschrieben.

Bei den erfindungsgemäßen Konstruktionsmaterialien ist das Trägermaterial mit einer Menge von 25 bis 75 Gew.-%, bevorzugt von 30 bis 70 Gew.-%, an wasserhärtender Polymerzubereitung, bezogen aus das gesamte Material, beschichtet und/oder imprägniert.

Es wurde auch ein Verfahren zur Herstellung wasserhärtender Polymerzubereitungen für Konstruktionsmaterialien gefunden, dadurch gekennzeichnet, daß man ein wasserhärtendes Reaktivharz mit einem Polysiloxan-Additiv der Formel

$R^1{}_3Si\text{-}O\text{-}[A]_m\text{-}SiR^1{}_3$ (I) und/oder 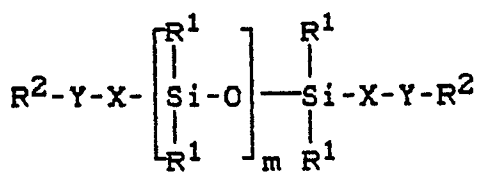 (II)

in der

$R^1$ für Niederalkyl steht,

m für die mittlere Zahl an Siloxangruppen im Bereich von 1 bis 20 steht
Y für einen Polyetherrest mit 1 bis 15 Ethylenoxid-Gruppen steht,
A für eine Polysiloxankette mit Strukturelementen der Formeln

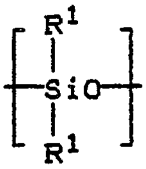 und/oder 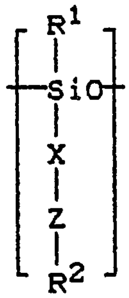 steht,

worin
$R^1$ die obengenannte Bedeutung hat,
X für einen Niederalkylenrest steht,
Z für einen Polyetherrest mit Ethylenoxid- und/oder Propylenoxid-Gruppen steht, wobei die mittlere Zahl der Ethylenoxid- und/oder Propylenoxid-Gruppen im Bereich von 10 bis 100 liegt, und
$R^2$ Wasserstoff und/oder Niederalkyl bedeuten,
wobei die Menge so gewählt ist, daß der kinetische Reibungskoeffizient nur 0,1 bis 0,3 geringer ist als ohne die entsprechenden Additive,
vermischt, Katalysatoren und weitere Hilfs- und Zusatzmittel zugibt und dann homogen über die Fläche des Trägermaterials verteilt.

Bei dem erfindungsgemäßen Verfahren arbeitet man unter Ausschluß von Feuchtigkeit. Bevorzugt wird bei einer relativen Feuchte <2 % (bei 21 °C), besonders bevorzugt bei <1 % (bei 21 °C), gearbeitet.

Für die Beschichtung bzw. Imprägnierung kann die Polymerzubereitung in einem inerten Lösungsmittel gelöst sein, das nach dem Beschichtungsvorgang wieder abgezogen wird.

Inerte Lösungsmittel können beispielsweise chlorierte Kohlenwasserstoffe, wie Methylenchlorid, Trichlorethan oder Chloroform, Ketone wie Aceton und Methylethylketon, Ester wie Essigsäureethylester und Butylacetat, Aromaten wie Toluol, Xylol oder entsprechende derivatisierte Typen, die keinen Zerewitinoff-aktiven Wasserstoff besitzen, sein.

Beispielsweise können die erfindungsgemäßen Konstruktionsmaterialien wie folgt hergestellt werden:
Man läßt das Trägermaterial über eine Walze laufen und tränkt es mit der Polymerzubereitung, gegebenenfalls in einem Lösungsmittel. Unmittelbar nach der Beschichtung bzw. Imprägnierung wird das Material in der gewünschten Länge (in der Regel 2 bis 11 m) auf geeignete Spulen aufgerollt und in einer luft- und wasserdichten Folie (z.B. aus Kunststoff-Aluminium-Laminat) oder anderen völlig dichten Behältern versiegelt, wie sie der DE-A-23 57 931, DE-A-26 51 089 und DE-A-30 33 569 beschrieben werden.

Unmittelbar vor der Anwendung wird das Material aus der Verpackung entnommen und um den betreffenden Gegenstand gewickelt oder anderweitig angebracht.

Für die Aushärtung werden die erfindungsgemäßen Zubereitungen mit Wasser, gegebenenfalls auch nur mit Luftfeuchtigkeit, in Kontakt gebracht.

Der Aushärtvorgang ist in der Regel kurz (etwa 3 bis 15 Minuten). In dieser Zeit sind die erfindungsgemäßen Zubereitungen überraschenderweise gut modellierbar und zeigen kaum Klebrigkeit.

Ihr kinetischer Reibungskoeffizient ist jedoch nur um 0,1 bis 0,3 geringer als der einer Zubereitung ohne entsprechende Menge Additiv.

Die erfindungsgemäßen Polymermodifikatoren, welche die Verarbeitung des beschichteten Trägermaterials wesentlich erleichtern, beeinflussen das Eigenschaftsbild der ausgehärteten Konstruktionsmaterialien in bezug auf Härte, Bruchdehnung und Lagenverbund nicht.

## Beispiele

### Beispiel 1

Herstellung eines erfindungsgemäßen wasserhärtenden Reaktivharzes (unter Verwendung des erfindungsgemäßen Additivs)

In einem 10 l Sulfiergefäß mit Edelstahl-Ankerrührer werden 6,5 kg Isocyanat (Bis-(4-isocyanatophenyl)-methan, welches carbodiimidisierte Anteile enthält [NCO-Gehalt = 29 %]), vorgelegt und auf ca. 50 °C vorgewärmt. Dazu werden 150 g eines UV-Stabilisators ( ein Cyanalkylindolderivat) zugegeben und so lange gerührt, bis der gesamte Feststoff gelöst ist. Nach Abkühlen auf Raumtemperatur werden 3,5 kg propoxylierten Triethanolamins (OH-Zahl = 150 mg KOH/g) innerhalb von 10 Minuten zugesetzt. Nach kurzem Temperaturanstieg auf 55 °C nach 55 Minuten fällt die Temperatur wieder und der Isocyanatgehalt erreicht nach 2 Stunden 13,4 %. Nach Zugabe von 534,2 g eines Polysiloxan-Polyoxyalkylen-Blockcopolymeren der Formel (I), welches 11,1 Gew.-% Siloxananteil und in den Polyether-Seitenketten 42,6 Gew.-% Ethylenoxid und 42,3 Gew.-% Propylenoxid, gestartet auf 4 Gew.-% n-Butanol enthält und ein mittleres Molekulargewicht von 1.850 g/Mol besitzt, wird das Reaktionsgemisch homogen gerührt, seine Viskosität liegt bei 19.160 mPa.s (25 °C).

Beispiel 2

Herstellung eines wasserhärtenden Reaktivharzes (Vergleichsbeispiel ohne Additiv)

In einem 10 l Sulfiergefäß mit Edelstahl-Ankerrührer werden 6,5 kg Isocyanat (Bis-(4-isocyanatophenyl)-methan, welches carbodiimidisierte Anteile enthält [NCO-Gehalt = 29 %]), vorgelegt und auf ca. 50 °C vorgewärmt. Dazu werden 150 g eines UV-Stabilisators ( ein Cyanalkylindolderivat) zugegeben und so lange gerührt, bis der gesamte Feststoff gelöst ist. Nach Abkühlen auf Raumtemperatur werden 3,5 kg propoxylierten Triethanolamins (OH-Zahl = 150 mg KOH/g) innerhalb von 10 Minuten zugesetzt. Nach kurzem Temperaturanstieg auf 55 °C nach 55 Minuten fällt die Temperatur wieder und der Isocyanatgehalt erreicht nach 2 Stunden 13,4 %. Der Isocyanatgehalt des fertigen Prepolymers beträgt 12,7 %, die Viskosität liegt bei 14.640 mPa.s (25 °C).

Beispiel 3

Herstellung eines erfindungsgemäßen wasserhärtenden Reaktivharzes (unter Verwendung des erfindungsgemäßen Additivs)

In einer Apparatur analog Beispiel 1 und 2 werden 6,48 kg Isocyanat (Bis-(4-isocyanatophenyl)-methan, welches carbodiimidisierte Anteile enthält [NCO-Gehalt = 29 %]) vorgelegt. Dann werden 7,8 g eines Polydime thylsiloxans mit $\eta_{25}$ = 30.000 mPa.s und 4,9 g Benzoylchlorid sowie danach 1,93 kg eines durch Propoxylierung von Propylenglykol hergestellten Polyethers (OH-Zahl = 112 mg KOH/g), 1,29 kg eines durch Propoxylierung von Glycerin hergestellten Polyethers (OH-Zahl = 250 mg KOH/g) und 190 g Dimorpholinodiethylether zugegeben. Nach 30 Minuten erreicht die Reaktionstemperatur 45 °C, nach 1 Stunde ist das Temperaturmaximum von 57 °c erreicht und der Isocyanatgehalt beträgt 14,2 %. 500 g eines Polysiloxan-Polyoxyalkylen-Blockcopolymeren, welches 11,1 Gew.-% Siloxananteil und in den Polyether-Seitenketten 42,6 Gew.-% Ethylenoxid und 42,3 Gew.-% Propylenoxid, gestartet auf 4 Gew.-% n-Butanol enthält und ein mittleres Molekulargewicht von 1.850 g/Mol besitzt, werden dann dem Reaktionsgemisch zugegeben und homogen verrührt. Der Isocyanatgehalt beträgt am Ende 13 %, die Viskosität liegt bei 20.200 mPa.s (25 °C).

Beispiel 4

Herstellung eines wasserhärtenden Reaktivharzes (Vergleichsbeispiel ohne Additiv)

In einer Apparatur analog Beispiel 1 und 2 werden 6,48 kg Isocyanat (Bis-(4-isocyanatophenyl)-methan, welches carbodiimidisierte Anteile enthält [NCO-Gehalt = 29 %]) vorgelegt. Dann werden, 7,8 g eines Polydimethylsiloxans mit $\eta_{25}$ = 30.000 mPa.s und 4,9 g Benzoylchlorid sowie danach 1,93 kg eines durch Propoxylierung von Propylenglykol hergestellten Polyethers (OH-Zahl = 112 mg KOH/g), 1,29 kg eines durch Propoxylierung von Glycerin hergestellten Polyethers (OH-Zahl = 250 mg KOH/g) und 190 g Dimorpholinodiethylether zugegeben.

Nach 30 Minuten erreicht die Reaktionstemperatur 42° C, nach 1 Stunde ist das Temperaturmaximum von 48° C erreicht und der Isocyanatgehalt beträgt 13,6 %. Die Viskosität liegt bei 17.800 mPas.

Beispiel 5

Zu einem Reaktivharz, der gemäß Beispiel 1 hergestellt wird, werden statt der 534,2 g des Polysiloxan-Polyoxyalkylen-Blockcopolymeren 534,2 g eines Polysiloxans der Formel

$$HO-(CH_2-CH_2-O)_{10}-(CH_2)_3-\underset{Me}{\overset{Me}{Si}}-(Me_2SiO)_{15}-\underset{Me}{\overset{Me}{Si}}-(CH_2)_3-(OCH_2CH_2)_{10}-OH$$

hinzugegeben und homogen verrührt.
Der Isocyanatgehalt beträgt dann 12,7 % und die Viskosität liegt bei 18 640 mPa.s (25° C).

Beispiel 6

Herstellung von Probeverbänden mit den Reaktivharzen der Beispiele 1 - 5

6a) Ein Glasfasergewirke (Breite 10,0 cm; Quadratmetergewicht ca. 290 g/m²), das in Längsrichtung eine Dehnung von ca. 25 % und in Querrichtung eine Dehnung von ca. 65 % aufweist (eine ausführliche Beschreibung dieser Glasfasergewirke findet sich in der US 4 609 578), wird mit 70 Gew.-% (bezogen auf Gewirke) des Harzes aus Beispiel 1 beschichtet. Die Beschichtung erfolgt in einer Atmosphäre, deren relative Feuchte durch einen Taupunkt des Wassers von unterhalb -20° C gekennzeichnet ist. Das Harz wird über ein geeignetes Walzenimprägnierwerk homogen auf das Gewirke aufgebracht. Eine mögliche geeignete Apparatur ist in der US 4 427 002 detailliert beschrieben. 3,66 m dieses beschichteten Produktes werden auf Kunststoffspulen von 1 cm Durchmesser aufgewickelt und in einer wasserdampfdurchlässigen Folie versiegelt.

6b) Analog zu Beispiel 6(a) wird das gleiche Glasfasergewirke mit 70 Gew.-% (bezogen auf Gewirke) des Harzes aus Beispiel 2 beschichtet und verpackt.

6c) Analog zu Beispiel 6(a) wird ein Polyestergewirke (Breite 10,0 cm, Quadratmetergewicht 118 g/m²), das in Längsrichtung eine Dehnung von ca. 55 % und in Querrichtung eine Dehnung von ca. 90 % aufweist und welches im Maschenstab ein polyfiles texturiertes Polyesterfilamentgarn (167 dtex, f 30x1) und in der Maschenreihe ein polyfiles hochfestes Polyesterfilamentgarn (550 dtex, f 96, normal schrumpfend) besitzt, mit 150 Gew.-% (bezogen auf Gewirke) des Harzes aus Beispiel 3 beschichtet und verpackt.

6d) Analog zu Beispiel 6(a) wird das Polyestergewirke des Beispiels 6(c) mit 150 Gew.-% (bezogen auf Gewirke) des Harzes aus Beispiel 4 beschichtet und verpackt.

6e) Analog zu Beispiel 6(a) wird das gleiche Glasfasergewirke mit 70 Gew.-% (bezogen auf Gewirke) des Harzes aus Beispiel 5 beschichtet und verpackt.

Beispiel 7

Vergleich der Abrollgeschwindigkeiten von unausgehärteten Probeverbänden mit wasserhärtenden Reaktivharzen gemäß Beispiel 1 und 2

Um ein Maß für die Leichtigkeit der Verarbeitung und für die gute Modellierbarkeit zu finden, wurden als einfacher Test die nach Beispiel 6 hergestellten Verbände nach 2 Monaten der Verpackung entnommen und die Kunststoffspule auf einer Stativstange drehbar gelagert aufgehängt. Durch ein am Ende des Verbandes befestigtes Gewicht von 1,6 N wurde dieser entrollt und die dafür erforderliche Zeit mit einer Stoppuhr ermittelt.

Nachfolgende Tabelle faßt die Ergebnisse zusammen:

| Probeverband | Abrollzeit [sec.] |
|---|---|
| Beispiel Nr. 6a | 15,4 |
| Beispiel Nr. 6b | 23,6 |

Beispiel 8

Bestimmung des kinetischen Reibungskoeffizienten der beschichteten Trägermaterialien 6(a) bis 6(e).

In Analogie zur EP 221 669 wurde entsprechend dem ASTM-D-1894-Test mit einer Apparatur der Fa. Instron Corp. (Instron Coefficient of Friction Fixture; Catalog No. 2810-005) und dem in der EP 221 669, Seite 13, beschriebenen Edelstahlschlitten der kinetische Reibungskoeffizient bestimmt.

Die Probeverbände wurde nach 1 Monat der Verpackung entnommen und wie in EP 221 660 Seite 14, 15, beschrieben, aufgearbeitet und vermessen. Die Kraftmessung erfolgte mit einer ZWICK Universal Prüfmaschine Typ 1484.

| Vergleichsproben: | Kin. Reibungskoeffizient |
|---|---|
| Scotchcast®2 (3M) (analog EP 221 669, Seite 17) | 2,0 |
| Scotchcast®Plus (3M) (Polydimethylsiloxangehalt: 4,7 %) | 0,3 |
| Beispiele (erfindungsgemäß) | |
| 6a | 1,5 |
| 6b | 1,6 |
| 6c | 0,6 |
| 6d | 0,8 |
| 6e | 1,4 |

## Ansprüche

1. Wasserhärtende Polymerzubereitung für Konstruktionsmaterialien, enthaltend als Additiv Polysiloxane der Formel

$$R^1_3Si-O-[A]_m-SiR^1_3 \quad \text{und/oder} \quad R^2-Y-X-\left[\;A\;\right]_m-\overset{\displaystyle R^1}{\underset{\displaystyle R^1}{Si}}-X-Y-R^2$$

(I) (II)

in der
$R^1$ für Niederalkyl steht,
m für eine mittlere Zahl von 1 bis 20 steht,
Y für einen Polyetherrest mit 1 bis 15 Ethylenoxid-Gruppen steht,
A für eine Polysiloxankette mit Strukturelementen der Formeln

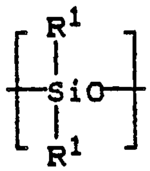 und/oder 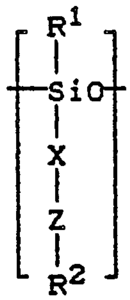 steht,

worin
$R^1$ die obengenannte Bedeutung hat,
X für einen Niederalkylenrest steht,
Z für einen Polyetherrest mit Ethylenoxid-und/ oder Propylenoxid-Gruppen steht, wobei die mittlere Zahl der Ethylenoxid-und/oder Propylenoxid-Gruppen im Bereich von 10 bis 100 liegt, und
$R^2$ Wasserstoff und/oder Niederalkyl bedeuten.

2. Wasserhärtende Polymerzubereitung nach Anspruch 1, enthaltend Polysiloxane der Formeln (I) und/oder (II)
wobei
$R^1$ für Methyl oder Ethyl steht,
m für die mittlere Zahl an Siloxangruppen im Bereich von 1 bis 10 steht,
Y für einen Polyetherrest mit 1 bis 15 Ethylenoxid-Gruppen steht,
A für eine Polysiloxankette mit Strukturelementen der Formeln

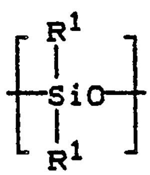 und/oder 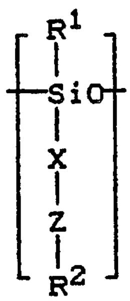 steht,

worin
$R^1$ die obengenannte Bedeutung hat,
X für Alkylen ($C_1$ bis $C_4$) steht,
Z für einen Polyetherrest mit Ethylenoxid-und Propylenoxid-Gruppen steht, wobei die mittlere Zahl der Ethylenoxid- und Propylenoxid-Gruppen im Bereich von 10 bis 100 liegt, und
$R^2$ Wasserstoff und/oder Alkyl ($C_1$ bis $C_4$) bedeutet,
wobei das Gewichts-Verhältnis der Ethylenoxid- zu Propylenoxid-Gruppen 20:80 % bis 80:20 % beträgt, wobei die Hydroxyl-Zahl im Bereich von 0 bis 80 [mgKOH/g] liegt.

3. Wasserhärtende Polymerzubereitung nach den Ansprüchen 1 und 2, enthalten Polysiloxane der Formeln (I) und/oder (II)
wobei
$R^1$ für Methyl oder Ethyl steht,
m für die mittlere Zahl an Siloxangruppen im Bereich von 1 bis 20 steht,
Y für einen Polyetherrest mit 2 bis 10 Ethylenoxid-Gruppen steht,
A für eine Polysiloxankette mit Strukturelementen der Formeln

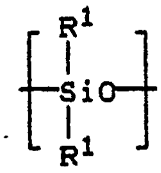 und/oder 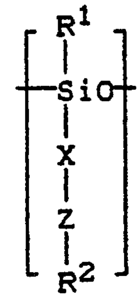 steht,

worin
$R^1$ die obengenannte Bedeutung hat,
X für Alkylen ($C_1$ bis $C_4$) steht,
Z für einen Polyetherrest mit Ethylenoxid-und Propylenoxid-Gruppen steht, wobei die mittlere Zahl der Ethylenoxid- und Propylenoxid-Gruppen im Bereich von 10 bis 100 liegt, und
$R^2$ Wasserstoff und/oder Alkyl ($C_1$ bis $C_4$) bedeutet,
wobei das Gewichts-Verhältnis der Ethylenoxid- zu Propylenoxid-Gruppen 20:80 % bis 80:20 % beträgt, wobei die Hydroxyl-Zahl im Bereich von 0 bis 80 [mgKOH/g] liegt.

4. Wasserhärtende Polymerzubereitungen nach den Ansprüchen 1 bis 3, enthaltend 0,1 bis 10 Gew.-% Polysiloxane bezogen auf das wasserhärtende Polymer.

5. Verfahren zur Herstellung von wasserhärtenden Polymerzubereitungen für Konstruktionsmaterialien, dadurch gekennzeichnet, daß man ein wasserhärtendes Reaktivharz mit einem Polysiloxan-Additiv der Formeln

$R^1{}_3Si-O-[A]_m-SiR^1{}_3$ und/oder 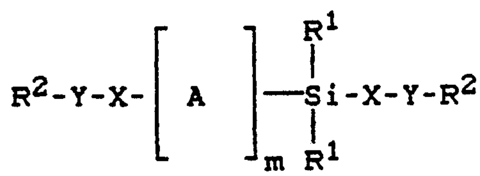

(I) (II)

in der
$R^1$ für Niederalkyl steht,
m für die mittlere Zahl an Siloxangruppen im Bereich von 1 bis 10 steht,
Y für einen Polyetherrest mit 1 bis 15 Ethylenoxid-Gruppen steht,
A für eine Polysiloxankette mit Strukturelementen der Formeln

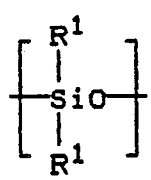 und/oder 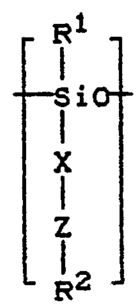 steht,

worin
$R^1$ die obengenannte Bedeutung hat,
X für einen Niederalkylenrest steht,
Z für einen Polyetherrest mit Ethylenoxid-und/ oder Propylenoxid-Gruppen steht, wobei die mittlere Zahl der Ethylenoxid-und/oder Propylenoxid-Gruppen im Bereich von 10 bis 100 liegt, und

$R^2$ Wasserstoff und/oder Niederalkyl bedeuten, vermischt, Katalysatoren und weitere Hilfs- und Zusatzmittel zugibt und dann homogen über die Fläche des Trägermaterials verteilt.

6. Verwendung von wasserhärtenden Polymerzubereitungen, enthaltend eine Polysiloxan-Additiv der Formel

$R^1{}_3Si\text{-}O\text{-}[A]_m\text{-}SiR^1{}_3$ und/oder 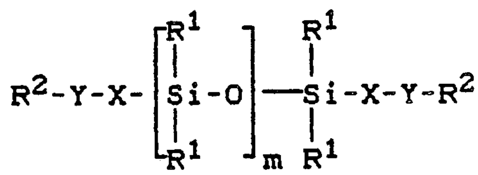

(I) (II)

in der
$R^1$ für Niederalkyl steht,
m für die mittlere Zahl an Siloxangruppen im Bereich von 1 bis 10 steht,
Y für einen Polyetherrest mit 1 bis 15 Ethylenoxid-Gruppen steht,
A für eine Polysiloxankette mit Strukturelementen der Formeln

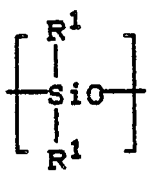 und/oder 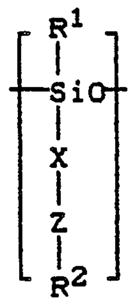 steht,

worin
$R^1$ die obengenannte Bedeutung hat,
X für Niederalkylenrest steht,
Z für einen Polyetherrest mit Ethylenoxid-und/ oder Propylenoxid-Gruppen steht, wobei die mittlere Zahl der Ethylenoxid-und/oder Propylenoxid-Gruppen im Bereich von 10 bis 100 liegt, und
$R^2$ Wasserstoff und/oder Niederalkyl bedeuten,
zur Beschichtung oder Imprägnierung von Trägern für Konstruktionsmaterialien.

7. Verwendung nach Anspruch 6, dadurch gekennzeichnet, daß als wasserhärtende Polymerzubereitung Polyisocyanate bzw. entsprechende Prepolymere mit mehr als zwei Isocyanatgruppen eingesetzt werden.

8. Verwendung nach den Ansprüchen 6 und 7, dadurch gekennzeichnet, daß medizinische Stützverbände hergestellt werden.

9. Verwendung nach den Anprüchen 6 und 7, daduch gekennzeichnet, daß technische Konstruktionsmaterialien hergestellt werden.